# EUROPEAN PATENT APPLICATION

(11) **EP 4 465 040 A1**
(43) Date of publication of application: **20.11.2024**
(21) Application number: 24176775.5
(22) Date of filing: 17.05.2024
(51) Int. Cl.: G01N 33/02, A01F 25/16

(54) **FLOATING MONITOR DEVICE FOR GRANULATES VOLUMES**

(30) Priority: 17.05.2023 PT 2023118657
(71) Applicant: INEGI - Instituto de Ciência e Inovação em Engenharia Mecânica e Engenharia, 4200-465 Porto (PT)
(72) Inventor: RIBEIRO DE SOUSA, Diogo Rui, 4200-518 Porto (PT); IDREIRA BARBOSA, José Carlos, 4460-663 Custóias (PT); FERREIRA MARTINS MACHADO, Silvino José, 4780-406 Santo Tirso (PT); DE LEMOS MIRANDA, Luís Filipe, 4475-662 Maia (PT); GERALDES TOURO PEREIRA, João Paulo, 4150-703 Porto (PT); CAETANO BAPTISTA, António José, 4465-103 S. Mamede Infesta (PT)
(74) Representative: Patentree

(57) **Abstract**

The present document discloses a floating monitor device for measuring chemical-physical parameters of a granulate volume, comprising: an electronic circuit comprising at least one sensor for transducing at least one chemical or physical parameter of the granulate, a data processor for capturing at least one transduced chemical-physical parameter, and an antenna for wirelessly transmitting at least one captured chemical-physical parameter; a granulate-tight and dust-tight casing enclosing said circuit. It is also disclosed a process for obtaining said floating monitor device and a system for measuring chemical-physical parameters of a granulate volume.

## Description

### TECHNICAL FIELD

The present disclosure relates to a floating monitoring device for measuring chemical and physical parameters inside storage, or transportation, of solid raw materials.

### BACKGROUND

For monitoring granulate storages, such as in grain silos, the use of hanging temperature-measuring cables is known. However, since these are static in relation with the flow of the cereals/granulates, they experience fast mechanical wear out, mainly during charging and discharging of the silo. Additionally, since these are placed inside of the silo, with limited access to the interior, maintenance and repairs are difficult and expensive. In horizontal storages, the movement of heavy machinery, such as wheel loaders or loading shovels is too aggressive, thus risking the ripping out these measuring cables while loading and unloading the granulate.

Manual probes are easy to deploy on horizontal storages, but usually can only go up to very shallow depths from the surface, and therefore are not helpful in monitoring vertical silos or big piles of granulate material.

Equipment near and around granulate storages are often required to be compliant with ATEX (*"ATmosphères EXplosibles*") regulations due to risk of explosion from suspended particles in air.

These facts are disclosed in order to illustrate the technical problem addressed by the present disclosure.

### GENERAL DESCRIPTION

The present document discloses a floating monitor device for measuring chemical-physical parameters of a granulate volume, comprising: an electronic circuit comprising at least one sensor for transducing at least one chemical or physical parameter of the granulate, a data processor for capturing at least one transduced chemical-physical parameter, and an antenna for wirelessly transmitting at least one captured chemical-physical parameter; a granulate-tight and dust-tight casing enclosing said circuit.

In an embodiment, the casing comprises a cylindrical body and a rounded shaped shell arranged on a first end of the cylindrical body, i.e., one of the bases of a cylinder.

In an embodiment, the device further comprises a battery for powering the electronic circuit and a receiving coil for inductively charging of the battery.

In an embodiment, the receiving coil is arranged on a second end of the cylindrical body, i.e., another of the bases of the cylinder, in particular the receiving coil being at least partially embedded in the casing. Further in particular, the receiving coil may be arranged between two parts of a casing cap arranged on an end of the cylindrical body, optionally two rounded shells being part of the same casing cap.

In an embodiment, the second end is flat or a rounded shaped shell which is flatter than the rounded shaped shell of the first end, for facilitating the wireless inductive connection to the receiving coil.

In an embodiment, the device has a density (relative density, kg/m³) which is approximately the same as the density of the granulate (i.e. the density being substantially the same as the density of the granulate, thus as required to achieve the effect of the device flowing with the granulate volume being measured as if it was part of the granulate volume), in particular the casing comprising an inner potting resin for providing a predetermined device density.

In an embodiment, the device has a density (relative density, kg/m³) which is within a range of -5% to +5% of the density of the granulate, in particular within a range of -2% to +2% of the density of the granulate, further in particular within a range of -1% to +1% of the density of the granulate.

In an embodiment, the casing is anti-electrostatic.

In an embodiment, the casing is made of a solid anti-electrostatic material or is made of a solid material coated with an anti-electrostatic material, in order to reduce the build-up of electrostatic charge and subsequent electrostatic discharge.

In an embodiment, the casing is made of a polymer, in particular nylon or polyethylene, further in particular an ultra-high molecular weight polyethylene.

In an embodiment, the granulate is: a cereal or a cereal mix comprising: rice, wheat, corn, oats, barley, quinoa, buckwheat, millet, rye, spelt, couscous, bulgur, polenta, tapioca, semolina, seeds, popcorn, or mixtures thereof; or a chemical granule or granulate comprising: food or chemical salts, polymers, plastics, wood pellets, fertilizers, glass beads, activated carbon, silica gel, or mixtures thereof; or a pharmaceutical granule or granulate comprising: active pharmaceutical ingredients, pharmaceutical excipients, or mixtures thereof.

In an embodiment, at least one measured chemical-physical parameter is selected from the group consisting of: concentration of volatile organic compounds in the air, concentration of carbon dioxide in the air, concentration of molecular oxygen in the air, temperature, moisture, pH, chemical toxin levels, molecular weight, or a combination of these.

In an embodiment, the antenna is at least partially embedded in the casing.

In an embodiment, the device further comprising a geopositioning receiver, preferably a satellite geopositioning receiver, for determining the location of the device.

In an embodiment, the device is larger than a predetermined sieve or filter size (i.e. a sieve or filter opening size) in order to be recoverable from the granulate volume (i.e. using said sieve or filter).

A process is also disclosed for obtaining said floating monitor device comprising the steps: placing the electronic circuit for transducing measurements within the casing, capturing, and transmitting at least one chemical or physical parameter of the granulate; closing the casing to make the device granulate-tight and dust-tight.

In an embodiment, the process further comprising the step of filing an inside volume of the casing with an electrical insulating potting resin.

A system for measuring chemical-physical parameters of a granulate volume is disclosed comprising the floating monitor device and a receiving station for receiving at least one captured chemical or physical parameter of the granulate, comprising: a system antenna to wirelessly receive data from the device; optionally, a local data storage for saving at least one measured chemical or physical parameter data.

In an embodiment, the system further comprising a concave shape for receiving the monitor device and emitter coil for inductively charging of the device, preferably with fitting pins for aligning the primary and the secondary coil.

### BRIEF DESCRIPTION OF THE DRAWINGS

The following figures provide preferred embodiments for illustrating the disclosure and should not be seen as limiting the scope of invention.
**Figures 1A****,** **1B and 1C****:** Schematic representation of an embodiment of a floating monitor device for measuring chemical-physical parameters of a granulate volume.
**Figure 2****:** Schematic representation of a vertical cross section of an embodiment of a casing of a floating monitor device.
**Figure 3****:** Schematic representation of an exploded view of an embodiment of a floating monitor device, showing the inductive charger coil, the thin cover on the induction end and the reinforcement for the thin section.
**Figures 4A****,** **4B, 4C****:** Schematic representation of an embodiment of a receiving station for receiving the at least one captured chemical or physical parameter of the granulate.

### DETAILED DESCRIPTION

The present document discloses a floating monitoring device for measuring chemical-physical parameters, at least partially immersed, inside a granulate storage, or container, e.g., a silo, comprising: at least one sensor for measuring at least one chemical-physical parameter; wireless transmission means for the transmission of the measured parameters; wherein the casing is coated with an anti-static material for reducing the build-up of electrostatic charge and subsequent electrostatic discharge.

For the sensing of chemical-physical parameters inside storage, the device is preferably fully immersed, thus being always in contact with storage content for a more accurate sensing of the chemical-physical parameters.

In an embodiment, the measured chemical-physical parameters are selected from the group comprising: the concentration of volatile organic compounds in the air, concentration of carbon dioxide in the air, concentration of molecular oxygen in the air, temperature, moisture, pH, chemical toxin levels (e.g. fermentation byproducts such as NO₂, Mycotoxins), molecular weight determination (e.g., via a mass spectrometer sensor), or a combination of these.

The density (relative density, km/m³) of the device is preferably the same or higher than the density of the granulate for being partially, or totally, immersed on the granulate material. Alternatively, the density of the device is approximately the same density of the granulate for flowing with the granulate volume as if part of the granulate volume.

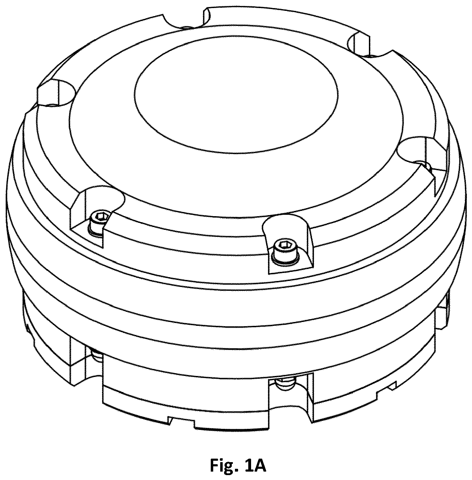

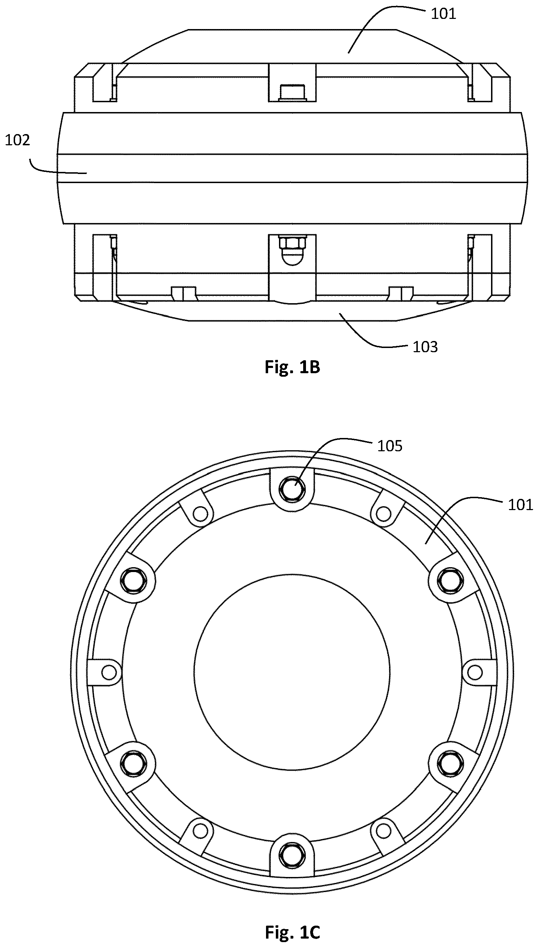

In **Figures 1A****,** **1B and 1C****,** an embodiment of a floating monitor device for measuring chemical-physical parameters of a granulate storage is shown, wherein 101 represents a top rounded shaped shell, 102 represents a cylindrical body, 103 represents a bottom rounded shaped shell, and 105 represents fastening means, preferably placed in a cavity of the casing.

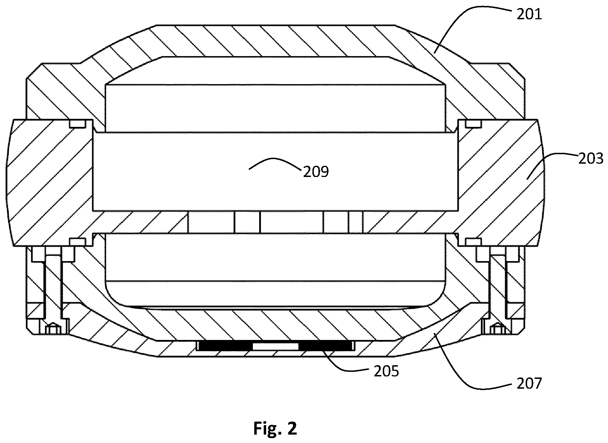

In **Figure 2****,** it is shown, in a vertical cross section, an embodiment of a casing of a floating monitor device, wherein 201 represents a first rounded shaped shell, 203 represents a cylindrical body, 205 represents a receiving coil, 207 represents a second rounded shaped shell with a flatter surface than the rounded shaped shell of a first rounded shaped shell, and 209 represents an housing for electronics and transmission means.

In an embodiment, the device is embedded in a casing comprising a cylindrical body and a rounded shaped shell arranged on both ends of the cylindrical body.

The rounded, roughly spherical geometry reduces the surface area, thus reducing the force exerted from the pressure on the device when it is buried in the granulate storage. The walls of the casing are also thick so that it can withstand impacts from an excavator shovel while the shovel is unloading material, e.g., grain from horizontal storage. Additionally, because of this rounded geometry, it is harder for the excavator shovel to unintentionally pierce it.

In an embodiment, the casing is a solid matrix made of a plastic material.

In an embodiment, the casing is made of a material with electrostatic discharge, e.g. in polymers a typical High Molecular Weight Polyethylene with Electrostatic Discharge, e.g., Polystone^{®} M ESD 90, can be used (or a polymer material, e.g., Nylon, coated with an anti-static material).

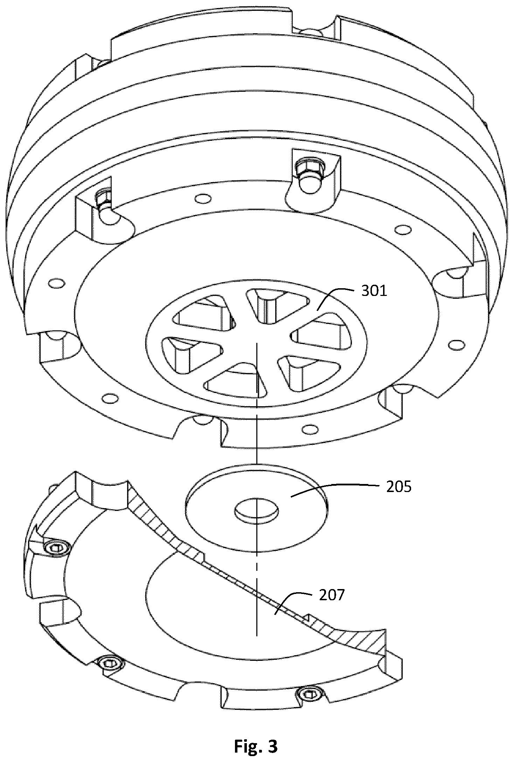

In **Figure 3** an embodiment of a floating monitor device is shown, in an exploded view, wherein 205 represents a receiving coil, 207 represents a second rounded shaped shell with a flatter surface than the rounded shaped shell of a first rounded shaped shell, and 301 represents reinforcing ribs.

In an embodiment, the monitoring device comprises an inductive charging transmitter coil, with a 12V input, and a receiver coil at the receiving station with a 5V output, capable of delivering 1.5A.

To ensure good and fast inductive charging, the coils have to be as close together as possible. In other words, the receiver coil inside the monitoring device has to be very close to the casing surface, which leads to very thin walls in a charging zone. However, the device has to be able to withstand strong impacts, e.g., from a loading shovel, so it is not ideal to have thin-walled areas. To solve this, the coil is placed in a thin-walled zone but is structurally reinforced from behind with a thicker wall.

In an embodiment, the wireless transmission means are at least partially comprised within said solid matrix.

In an embodiment, the wireless transmission means, e.g., an omnidirectional antenna, transmits the measured parameters to a remote server, preferably via a receiving station with a short/medium range of communication. In particular, the device comprises an antenna, namely a spiral antenna, for LoRa communication, e.g., at 433 MHz, for wirelessly transmitting the measured data.

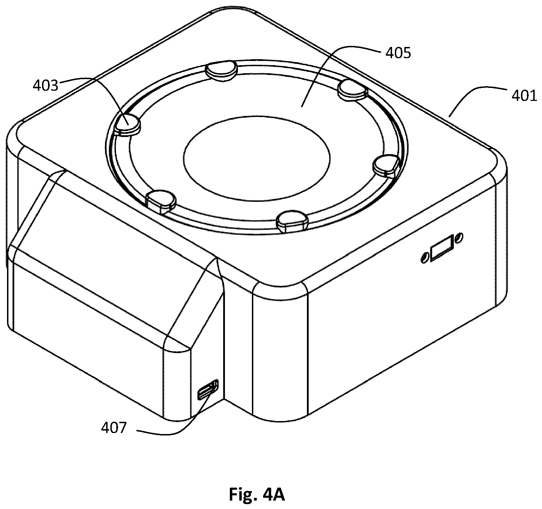

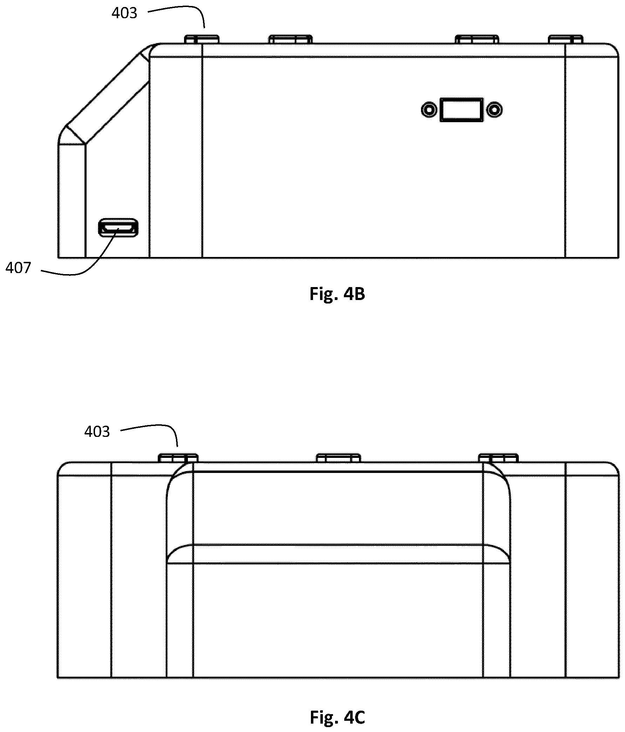

In **Figures 4A****,** **4B, 4C****,** an embodiment of a receiving station is shown, wherein 401 represents the receiving station, 403 represents fitting pins for ensuring the two coils are aligned, 405 represents a docking where the monitoring device is placed for charging, and 407 is a USB port for data access.

In an embodiment, the data is saved to an internal memory, and transmits it to a server over Wi-Fi, optionally, it could also connect over a wired protocol, i.e., Ethernet.

In an embodiment, the receiving station acts as a receptor for data coming from the monitoring device, and a transmitter of such data to a server, e.g., via a cellular or satellite data link.

In an embodiment, the receiving station further comprises means for inductively charging the monitoring device, e.g., using the Qi standard inductive charging, preferably comprising an inductive charging emitter coil and a docking portion based on 6 fitting pins. The pins guarantee proper alignment of the coils.

In an embodiment, the device further comprising a satellite positioning system, e.g., a GPS.

In an embodiment, the monitoring device is significantly larger in volume than the granulate material, making it possible to use a sieve or filter for recovering the device.

The assembly of the monitoring device comprises the following steps: placing at least one sensor for measuring at least one chemical-physical parameter inside a casing; filing the inside volume with an electrical insulating potting resin in order to prevent exposed electrical contacts and comply with safety requirements, e.g., ATEX requirements; closing the device with a second rounded shaped shell. To help with this sealing, and to ensure that there are no resin leaks at the filling time, i.e., when the resin is liquid, the casing openings are sealed with O-rings.

In an embodiment, the density of the device is calibrated via the resin density and the casing material density.

In an embodiment, the electrical insulating resin is an epoxy resin, thermosetting plastic, silicone rubber or other electronics encapsulation material.

The device is configured to be transported in a passive manner by the motion of a flow of said granulate materials. That is, when the granulate material is transferred, e.g., from a first granulate storage to a second granulate storage, the device remains inside the granulated material. This is particularly important to ensure that the monitoring of the chemical-physical parameters before, during and after the granulate transference are not interrupted.

To ensure that the insulating resin reaches and covers the coil, this reinforcement has perforations that allow the resin to pass through, and results in a ribbing.

In an embodiment, the storage comprises solid raw materials in the form of powder, granulates, pellets, or a mixture comprising a subset of these. For example, cereals, rice, wheat, corn, oats, barley, quinoa, buckwheat, millet, rye, spelt, couscous, bulgur, polenta, tapioca, semolina, seeds, popcorn.

The device comprises the following advantages: high wear resistance, anti-static properties, water-tightness, and high resistance to compressive forces/pressure.

For example, when unloading inside a silo, the device experiences high friction surface stresses due to the grain rubbing against the surface of the device in a highpressure environment. It is therefore important to ensure that the casing is made of a material that does not deteriorate within a few operating cycles or that could contaminate the content with scraps.

Additionally, it must be ensured that there is no build-up of static surface charges on the device that could lead to sparks. Thus, ensuring that the electrostatic surface charge is reduced, dust is not attracted, and no electrostatic discharge occurs.

One of the advantages of the disclosed monitoring device is the possibility to continuously monitor the respective batch of grains, both in a vertical silo or horizontal storage, and during transportation. Thus, tracking the grain along the distributing chain.

It also allows volumetric measurement of the parameters being measured, because measurements with a manual probe are superficial and the hanging temperature-measuring cables in the silos are subject to considerable wearing forces and have low mechanical reliance.

The term "comprising" whenever used in this document is intended to indicate the presence of stated features, integers, steps, components, but not to preclude the presence or addition of one or more other features, integers, steps, components, or groups thereof.

The disclosure should not be seen in any way restricted to the embodiments described and a person with ordinary skill in the art will foresee many possibilities to modifications thereof. The above-described embodiments are combinable.

The following claims further set out particular embodiments of the disclosure.

## Claims

1. Floating monitor device for measuring chemical-physical parameters of a granulate volume, comprising:
an electronic circuit comprising at least one sensor for transducing at least one chemical or physical parameter of the granulate, a data processor for capturing at least one transduced chemical-physical parameter, and an antenna for wirelessly transmitting at least one captured chemical-physical parameter;
a granulate-tight and dust-tight casing enclosing said circuit; and
a battery for powering the electronic circuit and a receiving coil for inductively charging of the battery;
wherein the receiving coil is arranged on an end of a cylindrical body, in particular the receiving coil being at least partially embedded in the casing;
wherein the casing comprises a cylindrical body.

2. Device according to any of the previous claims wherein the device has a density which is approximately the same as the density of the granulate, in particular the casing comprising an inner potting resin for providing a predetermined device density.

3. Device according to the previous claim wherein the device has a density which is within a range of -5% to +5% of the density of the granulate, in particular within a range of -2% to +2% of the density of the granulate, further in particular within a range of -1% to +1% of the density of the granulate.

4. Device according to the previous claim further wherein said end is a second end and wherein the casing comprises a rounded shaped shell arranged on a first end of the cylindrical body.

5. Device according to any of the previous claim wherein the second end is flat or a rounded shaped shell which is flatter than the rounded shaped shell of the first end.

6. Device according to any of the previous claims wherein the casing is anti-electrostatic, preferably the casing is made of a solid anti-electrostatic material or is made of a solid material coated with an anti-electrostatic material.

7. Device according to any of the previous claims wherein the casing is made of a polymer, in particular nylon or polyethylene, further in particular an ultra-high molecular weight polyethylene.

8. Device according to any of the previous claims wherein the granulate is:
a cereal or a cereal mix comprising: rice, wheat, corn, oats, barley, quinoa, buckwheat, millet, rye, spelt, couscous, bulgur, polenta, tapioca, semolina, seeds, popcorn, or mixtures thereof; or
a chemical granule or granulate comprising: food or chemical salts, polymers, plastics, wood pellets, fertilizers, glass beads, activated carbon, silica gel, or mixtures thereof; or
a pharmaceutical granule or granulate comprising: active pharmaceutical ingredients, pharmaceutical excipients, or mixtures thereof.

9. Device according to any of the previous claims wherein at least one measured chemical-physical parameter is selected from the group consisting of: concentration of volatile organic compounds in the air, concentration of carbon dioxide in the air, concentration of molecular oxygen in the air, temperature, moisture, pH, chemical toxin levels, molecular weight, or a combination of these.

10. Device according to any of the previous claims wherein the antenna is at least partially embedded in the casing and/or further comprising a geopositioning receiver, preferably a satellite geopositioning receiver, for determining a location of the device.

11. Device according to any of the previous claims wherein the device is larger than a predetermined sieve or filter size for being recoverable from the granulate volume.

12. Process for obtaining a floating monitor device according to any of the previous claims comprising the steps:
placing within the casing the electronic circuit for transducing, capturing and transmitting at least one chemical or physical parameter of the granulate;
closing the casing to make the device granulate-tight and dust-tight.

13. Process according to the previous claim comprising the step of filing an inside volume of the casing with an electrical insulating potting resin.

14. System for measuring chemical-physical parameters of a granulate volume comprising the floating monitor device according to any of the claims 1-11 and a receiving station for receiving at least one captured chemical or physical parameter of the granulate, comprising:
a system antenna to wirelessly receive data from the device;
optionally, a local data storage for saving at least one measured chemical or physical parameter data.

15. System according to the previous claim further comprising a concave shape for receiving the monitor device and emitter coil for inductively charging of the device, preferably with fitting pins for aligning the primary and the secondary coil.
